# EUROPÄISCHE PATENTANMELDUNG

(11) **EP 0 564 841 A1**
(43) Veröffentlichungstag der Anmeldung: **13.10.1993**
(21) Anmeldenummer: 93104020.8
(22) Anmeldetag: 12.03.1993
(51) Int. Cl.: C07D 207/333

(54) **Verfahren zur Herstellung von Pyrrolderivaten**

(30) Priorität: 06.04.1992 DE 4211531
(71) Anmelder: CASSELLA Aktiengesellschaft, D-60386 Frankfurt (DE)
(72) Erfinder: Zoller, Gerhard, Dr., W-6269 Schöneck (DE); Kujath, Eckhard, Dr., W-6457 Maintal 1 (DE); Delpy, Klaus, Dr., W-6057 Dietzenbach (DE); Schrod, Manfred, Dr., W-6108 Weiterstadt (DE)
(74) Vertreter: Muley, Ralf, Dr.

(57) **Zusammenfassung**

Die vorliegende Erfindung betrifft ein Verfahren zur Herstellung von Pyrrolderivaten der allgemeinen Formel I 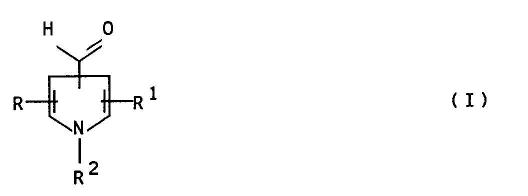
durch Umsetzung einer Verbindung der allgemeinen Formel III 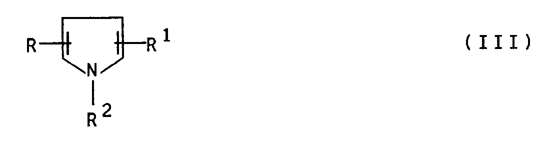
wobei R, R¹ und R² wie in Anspruch 1 angegeben definiert sind, in einer Vilsmeier-Reaktion, wobei die Umsetzung in einem leicht abbaubaren Lösungsmittel ausgeführt, das Reaktionsprodukt mit Hilfe eines wassermischbaren Extraktionsmittels isoliert und durch Hochvakuumdestillation im Kurzwegverdampfer gereinigt wird.

## Beschreibung

Die vorliegende Erfindung betrifft ein Verfahren zur Herstellung von Pyrrolaldehyden durch Vilsmeier-Synthese und Reinigung durch Hochvakuumdestillation. Pyrrolaldehyde sind wertvolle pharmakologisch wirksame Verbindungen und beispielsweise in der EP-A 287 988 beschrieben. Dort ist auch bereits eine Vilsmeier-Synthese zu ihrer Herstellung angegeben.

Die Synthese größerer Substanzmengen nach diesem Verfahren bereitet allerdings Probleme hinsichtlich der technischen Durchführbarkeit. Außerdem sind im technischen Maßstab die Ausbeuten relativ niedrig und die Reinheiten der Produkte entsprechen nicht den Ansprüchen, die an einen pharmakologischen Wirkstoff gestellt werden müssen. Da bei der Reaktion selbst große Mengen an halogenhaltigen Lösungsmitteln eingesetzt werden müssen, gibt es darüberhinaus erhebliche Probleme bei der Abwasser- und Abluftreinhaltung, sowie hinsichtlich der allgemeinen Arbeitssicherheit. Die Reinigung der Produkte durch mehrmalige Umkristallisation ist mit erheblichen Ausbeuteverlusten und Einsatz großer Lösungsmittelmengen verbunden.

Aufgabe vorliegender Erfindung ist deshalb, ein Verfahren bereitzustellen, das Pyrrolaldehyde auch im technischen Maßstab in guten Ausbeuten und den erforderlichen Reinheiten liefert und das zudem ökologisch möglichst einwandfrei ist.

Diese Aufgabe wird überraschend gelöst durch ein Verfahren zur Herstellung von Pyrrolderivaten der allgemeinen Formel I 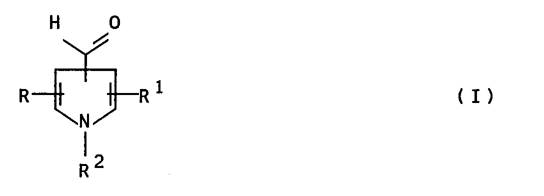
worin R und R¹ unabhängig voneinander Wasserstoff oder (C₁-C₄)-Alkyl;
R² (C₁-C₃)-Alkyl, das durch -NH₂, Acylamino der allgemeinen Formel II 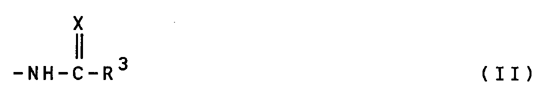
Formyl, Mono- oder Di-(C₁-C₄)-alkoxyethyl, (C₁-C₄)-Alkoxycarbonyl oder Cyano substituiert ist;
X ein Sauerstoff- oder Schwefelatom; und
R³ Wasserstoff, gegebenenfalls substituiertes (C₁-C₅)-Alkyl, (C₅-C₇)-Cycloalkyl, gegebenenfalls substituiertes Phenyl oder Phenylamino, Amino oder gegebenenfalls substituiertes (C₁-C₅)-Alkylamino
bedeutet, durch Umsetzung einer Verbindung der allgemeinen Formel III 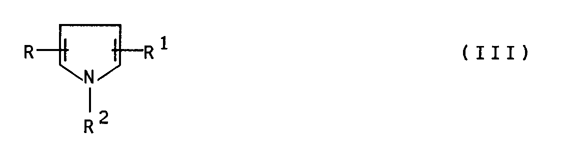
worin R, R¹ und R² wie oben angegeben definiert sind in einer Vilsmeier-Reaktion, dadurch gekennzeichnet, daß die Umsetzung in einem leicht abbaubaren Lösungsmittel ausgeführt, das Reaktionsprodukt mit Hilfe eines wassermischbaren Extraktionsmittels isoliert und durch Hochvakuumdestillation im Kurzwegverdampfer gereinigt wird.

In den vorstehenden Definitionen genannte Alkyl-, Alkoxy- und Alkylaminogruppen können geradkettig oder verzweigt sein.
Alkylgruppen sind beispielsweise Methyl, Ethyl, Propyl und Butyl. Entsprechendes gilt für Alkoxy und Alkylamino. (C₅-C₇)-Cycloalkyl ist insbesondere Cyclopentyl, Cyclohexyl und Cycloheptyl.
Die Formylgruppe kann in der 2- oder 3-Position des Pyrrolrings stehen.
R und R¹ bedeuten bevorzugt Wasserstoff oder Methyl.
X bedeutet bevorzugt Sauerstoff.
Für R³ stehendes Alkyl oder Alkylamino hat bevorzugt 1 oder 2 C-Atome. Es kann gegebenenfalls durch Amino, Mono- oder Di-(C₁-C₄)alkylamino oder Phenoxy substituiert sein. Für R³ stehendes Phenyl oder Phenylamino kann mit bis zu drei Substituenten substituiert sein. Geeignete Substituenten sind insbesondere Chlor, (C₁-C₂)-Alkyl, (C₁-C₂)-Alkoxy, (C₁-C₂)-Alkoxycarbonyl und Carboxyl.
Bevorzugte Reste R² sind (C₁-C₃)-Alkylrest, die durch Acetylamino, Propionylamino, Isopropionylamino, 4-Chlorbenzoylamino, 4-Chlorphenylaminocarbonylamino, Cyanopropyl oder 2,2-Diethoxyethyl substituiert sind.

Das erfindungsgemaße Verfahren kann mit allen in der Vilsmeier-Synthese üblichen und literaturbekannten Reagenzien ausgeführt werden (Houben-Weyl, Methoden der organischen Chemie, 4. Auflage, E 3, S. 16ff, 1983). Bevorzugt ist aber das Verfahren, das mit Dimethylformamid und Phosphoroxychlorid arbeitet. Die Hydrolyse des Vilsmeier-Komplexes geschieht ebenfalls in bekannter Weise, beispielsweise mit Natronlauge.

Bevorzugte leicht abbaubare Lösungsmittel, in denen das erfindungsgemäße Verfahren ausgeführt wird, sind z. B. 1,2-Dimethoxyethan, Tetrahydrofuran, Dioxan und Diethylenglykoldimethylether.

Bei Einsatz von beispielsweise 6 normaler Natronlauge zur Hydrolyse des Vilsmeier-Komplexes kann der Anteil an 1,2-Dimethoxyethan im Bereich von 10 bis über 100 Vol%, bezogen auf die Natronlauge variiert werden, wobei ein Anteil von 40 bis 80 Vol% besonders bevorzugt ist.

Bevorzugte wassermischbare Extraktionsmittel, die zur Isolierung der Pyrrolderivate verwendet werden, sind 1,2-Dimethoxyethan und Tetrahydrofuran.

In einer besonders bevorzugten Ausführungsform des erfindungsgemäßen Verfahrens wird bei der Hydrolyse des Vilsmeier-Komplexes das Reaktionsgemisch über eine externe Kühleinrichtung im Kreislauf umgepumpt.

Dadurch gelingt es, eine aufgrund der sehr stark exothermen Reaktion zu starke Erwärmung des Reaktionsgemisches zu vermeiden und somit den Anteil an Nebenprodukten zu minimieren.

Durch Hochvakuumdestillation im Kurzwegverdampfer bei sehr gutem Vakuum ist es gelungen, die empfindlichen Pyrrolderivate ohne Zersetzung in guter Ausbeute und Reinheit zu isolieren.

Das erfindungsgemäße Verfahren liefert auch im technischen Maßstab Pyrrolderivate der allgemeinen Formel I in guten Ausbeuten und hoher Reinheit und ist dabei ökologisch verträglich. Es ist damit den bekannten Herstellungsverfahren in jeder Hinsicht erheblich überlegen und stellt eine nicht vorhersehbare Bereicherung der Technik dar.

### Beispiel 1:

### Essigsäure-N-(2-(3-formyl-2,5-dimethyl-1H-pyrrolyl)ethyl) -amid

Zu 750 ml 1,2-Dimethoxyethan und 109 ml Dimethylformamid werden bei 0 ^{o}C 123 ml (1,34 mol) Phosphoroxychlorid innerhalb von 30 Minuten zugetropft. Man rührt 30 Minuten bei 0 ^{o}C nach trägt innerhalb von 45 Minuten bei 0 ^{o}C 180 g (1 mol) Essigsäure-N-(2-(2,5-dimethyl-1H-pyrrolyl)ethyl)-amid ein. Nach 10 Minuten bei 0 ^{o}C läßt man auf 10 ^{o}C erwärmen, rührt 1 h bei 10 ^{o}C und hydrolysiert durch Eingießen einer Lösung aus 250 g Natriumhydroxid in 920 ml Wasser innerhalb von 2 h bei 20 ^{o}C in das Reaktionsgemisch. Das erhaltene Reaktionsgemisch wird filtriert, die organische Phase abgetrennt und die Wasserphase zweimal mit je 370 ml 1,2-Dimethoxyethan extrahiert. Die organischen Phasen werden vereinigt und im Vakuum weitgehend eingeengt. Nach Zugabe von 250 ml Toluol wird zum Entwässern eingeengt, mit 1 l Essigester versetzt, über Kieselgel und Kohle filtriert, kristallisiert und getrocknet.
- Ausbeute:: 166 g (80 %)
- Schmelzpunkt:: 115 - 117 ^{o}C
Zur weiteren Reinigung wurde das Rohprodukt in einer Hochvakuumapparatur bei einem Vakuum von << 10⁻³ mbar und einer Badtemperatur von 220 - 240 ^{o}C destilliert und aus 360 ml 1,2-Dimethoxyethan umkristallisiert.
Ausbeute: 155 g (74,7 %)
Schmelzpunkt: 118 - 119 ^{o}C
Siedepunkt: 190 - 195 ^{o}C / < 10⁻³ mbar
Reinheit: > 99,9 % (HPLC, DSC)

| Elementaranalyse: C₁₁H₁₆N₂O₂ (208,26) | | | | |
|---|---|---|---|---|
| Berechnet: | C 63,4 | H 7,7 | N 13,5 | O 15,4 |
| Gefunden: | C 63,3 | H 7,7 | N 13,5 | O 15,4 |

Der gleiche Versuchsablauf wurde im 30-fachen Maßstab und entsprechendem Ergebnis durchgeführt.

### Beispiel 2:

Analog Beispiel 1 wurde die Umsetzung in Tetrahydrofuran als Lösungsmittel durchgeführt.
- Ausbeute:: 77 %

### Beispiel 3:

Analog Beispiel 1 wurde die Umsetzung in Diethylenglykoldimethylether als Lösungsmittel durchgeführt.
- Ausbeute:: 70 %

### Beispiel 4:

### 1-(2,2-Diethoxyethyl)-2,5-dimethylpyrrol-3-aldehyd

Die Verbindung wurde analog Beispiel 1 durch Formylierung von 1-(2,2-Diethoxyethyl)-2,5-dimethylpyrrol erhalten und durch Hochvakuumdestillation gereinigt.
- Schmelzpunkt:: 109 - 111 ^{o}C
- Siedepunkt:: 175 - 180 ^{o}C / < 10⁻³ mbar

## Patentansprüche

1. Verfahren zur Herstellung von Pyrrolderivaten der allgemeinen Formel I 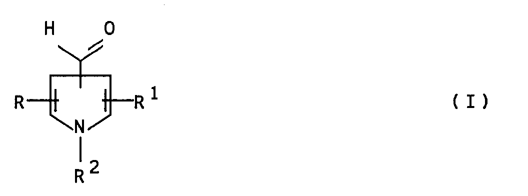 worin R und R¹ unabhängig voneinander Wasserstoff oder (C₁-C₄)-Alkyl;
R² (C₁-C₃)-Alkyl, das durch -NH₂, Acylamino der allgemeinen Formel II 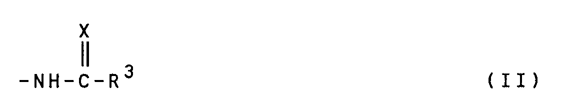 Formyl, Mono- oder Di-(C₁-C₄)-alkoxymethyl, (C₁-C₄)-Alkoxycarbonyl oder Cyano substituiert ist;
X ein Sauerstoff- oder Schwefelatom; und
R³ Wasserstoff, gegebenenfalls substituiertes (C₁-C₅)-Alkyl, (C₅-C₇)-Cycloalkyl, gegebenenfalls substituiertes Phenyl oder Phenylamino, Amino oder gegebenenfalls substituiertes (C₁-C₅)-Alkylamino
bedeutet, durch Umsetzung einer Verbindung der allgemeinen Formel III 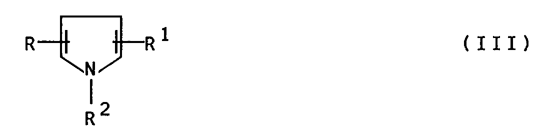 worin R, R¹ und R² wie oben angegeben definiert sind, in einer Vilsmeier-Reaktion, dadurch gekennzeichnet, daß die Umsetzung in einem leicht abbaubaren Lösungsmittel ausgeführt, das Reaktionsprodukt mit Hilfe eines wassermischbaren Extraktionsmittels isoliert und durch Hochvakuumdestillation im Kurzwegverdampfer gereinigt wird.

2. Verfahren gemäß Anspruch 1, dadurch gekennzeichnet, daß R und R¹ unabhängig voneinander Wasserstoff oder Methyl bedeuten.

3. Verfahren gemäß Anspruch 1 und/oder 2, dadurch gekennzeichnet, daß X Sauerstoff bedeutet.

4. Verfahren gemäß einem oder mehreren der Ansprüche 1 bis 3, dadurch gekennzeichnet, daß R² (C₁-C₃)-Alkyl bedeutet, das durch Acetylamino, Propionylamino, Isopropionylamino, 4-Chlorbenzoylamino, 4-Chlorphenylamino-carbonylamino, Cyanopropyl oder 2,2-Diethoxyethyl substituiert ist.

5. Verfahren gemäß einem oder mehreren der Ansprüche 1 bis 4, dadurch gekennzeichnet, daß die Vilsmeier-Reaktion mit Dimethylformamid und Phosphoroxychlorid ausgeführt wird.

6. Verfahren gemäß einem oder mehreren der Ansprüche 1 bis 5, dadurch gekennzeichnet, daß als Lösungsmittel 1,2-Dimethoxyethan, Tetrahydrofuran oder Diethylenglykoldimethylether eingesetzt werden.

7. Verfahren gemäß einem oder mehreren der Ansprüche 1 bis 6, dadurch gekennzeichnet, daß als Extraktionsmittel 1,2-Dimethoxyethan oder Tetrahydrofuran eingesetzt werden.

8. Verfahren gemäß einem oder mehreren der Ansprüche 1 bis 7, dadurch gekennzeichnet, daß bei der Hydrolyse des Vilsmeier-Komplexes das Reaktionsgemisch über eine externe Kühleinrichtung im Kreislauf umgepumpt wird.
